(19) Europäisches Patentamt
European Patent Office
Office européen des brevets

(11) **EP 0 878 549 B1**

(12) **EUROPÄISCHE PATENTSCHRIFT**

(45) Veröffentlichungstag und Bekanntmachung des Hinweises auf die Patenterteilung:
**07.03.2007 Patentblatt 2007/10**

(51) Int Cl.:
*C12P 19/18* *(2006.01)*        *C12P 19/44* *(2006.01)*
*C12N 9/10* *(2006.01)*        *C08B 37/00* *(2006.01)*
*A61K 8/60* *(2006.01)*        *A61K 31/70* *(2006.01)*

(21) Anmeldenummer: **98106603.8**

(22) Anmeldetag: **09.04.1998**

(54) **Verfahren zur enzymatischen Herstellung von Fructosylverbindungen**

Process for the enzymatic production of fructosyl compounds

Procédé pour la fabrication enzymatique de composés du fructosyle

(84) Benannte Vertragsstaaten:
**AT BE CH DE ES FR GB IT LI NL**

(30) Priorität: **14.04.1997 DE 19716731**

(43) Veröffentlichungstag der Anmeldung:
**18.11.1998 Patentblatt 1998/47**

(73) Patentinhaber: **Chemisches Laboratorium Dr. Kurt Richter GmbH**
**12159 Berlin (DE)**

(72) Erfinder:
• **Ringpfeil, Manfred, Prof. Dr.**
**12621 Berlin (DE)**
• **Pelenc, Vincent, Dipl.-Ing.**
**30451 Hannover (DE)**
• **König, Joachim, Dr.**
**13053 Berlin (DE)**
• **Petersen, Rolf-Dieter, Dr.**
**14199 Berlin (DE)**
• **Borchert, Stefan, Dr.**
**12209 Berlin (DE)**

(74) Vertreter: **Vossius & Partner**
**Siebertstrasse 4**
**81675 München (DE)**

(56) Entgegenhaltungen:
**EP-A- 0 447 125          WO-A-91/16449**
**WO-A-95/02683          WO-A-98/33492**
**GB-A- 2 182 558**

• **DATABASE WPI Section Ch, Week 199111 Derwent Publications Ltd., London, GB; Class D16, AN 1991-078668 XP002144094 & JP 03 027285 A (ENSUIKO SUGAR REFINING CO LTD), 5. Februar 1991 (1991-02-05)**
• **HERNANDEZ ET AL.: "Isolation and enzymic properties of levansucrase secreted by Acetobacter diazotrophicus SRT4." BIOCHEMICAL JOURNAL, Bd. 109, 1995, Seiten 113-118, XP000917365**

**Beschreibung**

Fructosyltransferasen katalysieren den Transfer von Fructosylgruppen aus Saccharose zu einem Akzeptor. Die Reaktion verläuft gemäß dem folgenden Schema:

```
Saccharose                                              Glucose
                                                            `

        +                         ⇒                           +


Akzeptor        Fructosyltransferase        Fructosyl-Akzeptor
```

Diese Transferreaktion besitzt eine doppelte Spezifizität bezüglich des Fructosyldonators und des Akzeptors von Fructosylgruppen. Der übliche Donator von Fructosylgruppen ist die Saccharose. Weitere bekannte Fructosyldonatoren sind Saccharoseanaloga, in denen die Glucose-Hälfte der Saccharose durch Monosaccharide, wie $\alpha$-D-Galactose, $\beta$-L-Arabinose, $\alpha$-D-Xylose, oder durch Disaccharide, wie Lactose, Maltose, Isomaltose, Melibiose, oder durch Oligosaccharide, wie Oligodextrane oder Maltodextrine, ersetzt worden ist (Hestrin und Avigad, 1958, Biochem., J., Vol. 69, pp. 388-398).

Ein bekannter Akzeptor der Reaktion ist die Sacccharose. Die Fructosylierung von Saccharose führt zu Oligosacchariden und Polysacchariden (Avigad und Feingold, 1957, Arch. Biochem. Biophys., Vol, 70, pp. 178-184).

Glucose wird bei der Reaktion freigesetzt und wirkt ebenfalls neben Saccharose als Akzeptor von Fructosylgruppen. Glucoseanaloga, wie $\alpha$-D-Galactose, $\beta$-L-Arabinose, $\alpha$-D-Xylose, oder Disaccharide, wie Lactose, Maltose, Isomaltose, Melibiose, oder Oligosaccharide, wie Oligodextrane oder Maltodextrine, können zu Saccharoseanaloga fructosyliert werden. Diese Reaktion wurde zur Herstellung von Xylosylfructosid und Lactosylfructosid beschrieben (Hayashibara, EP 0 447 125).

JP 03 027285 A offenbart die Kultivierung von Mikroorganismen, die zu den Arthrobactern gehören um eine neue beta-Fructosidase zu erhalten, die eine zuckerübertragende Wirkung hat.

Hernandez et al., Biochemical Journal, Bd. 109, 1995, Seiten 113-118 beschreibt die Isolierung und die enzymatischen Eigenschaften von Levansaccharase, welche von *Acetobacter diazotrophicus* SRT4. sezerniert wird.

GB 2182558 beschreibt Arzneimittel zur Vorbeugung, welche ein Fructo-Oligosaccharid enthalten.

WO 95/02683 A und WO 91/16449 A offenbaren, dass Saccharidmoleküle aus Nukleosiden, Mono- bzw. Diphosphate mittels entsprechenden zelloberflächengebundenen Glycosyltransferasen aus Wirbeltieren bzw. Pflanzen an unterschiedliche Akzeptoren angekoppelt werden können.

WO 98/33492 A, ein nachveröffentlichtes Dokument, welches nur zur Beurteilung der Neuheit herangezogen wird, beschreibt eine Klasse von Enzyminhibitoren, die die Konversion von Fructose-lysin (FL) zu Fructose-lysin-3-phosphat hemmen.

Wasser wirkt auch als Akzeptor der Reaktion, was zur Freisetzung von Fructose führt. Es ist weiter bekannt, daß Wasseranaloga, wie aliphatische kurzkettige Alkohole (Methanol, Ethanol), und Polyole, wie Glycerol und Sorbitol, als Akzeptoren wirken können. Die bisher bekannten Akzeptoren waren jedoch die natürlichen Akzeptoren und ihre Analoga und führten zu Fructosylverbindungen, die in ihrer Anwendung begrenzt waren.

Es wurde nun gefunden, daß überraschenderweise andere Substanzen als die bisher bekannten als Akzeptoren von Fructosylgruppen dienen können. Dies ermöglicht den Einsatz von viel nützlicheren Akzeptoren als die bisher bekannten und dadurch die Bildung von Fructosylverbindungen, die breiter angewendet werden können. Man war bisher davon ausgegangen, daß das spezifische Enzym Fructosyltransferase nur natürliche Akzeptoren und deren Analoga fructosylieren kann. Die vorliegende Erfindung überwindet dieses Vorurteil und stellt ein neues Verfahren zur Herstellung von Fructosylverbindungen mit breiter Anwendungsmöglichkeit aus bisher nicht eingesetzten Akzeptoren zur Verfügung.

Zur Herstellung von Fructosylverbindungen im Sinne der Erfindung wird ein Reaktionsgemisch aus Saccharose und Akzeptor der Enzymwirkung einer Fructosyltransferase ausgesetzt.

Der erfindungsgemäße Akzeptor ist eine Verbindung, die anwendungsgemäß eine besonders gewünschte biologische Wirkung besitzt, wobei Zucker mit 4 oder mehr Kohlenstoffatomen, die ausschließlich Alkohol-, Aldehyd-, Keto-, oder Hemiacetalfunktionen enthalten, sowie C1-C6 aliphatische Verbindungen, die als funktionelle Gruppe ausschließlich eine oder mehrere OH-Gruppen besitzen, als Akzeptoren ausgeschlossen sind.

Substanzen, wie Vitamine, Hormone, neurologische Botenstoffe, sekundäre Metabolite aus Pflanzen oder Mikroorganismen, Aminosäuren, Peptide, Proteine, Hydroxysäuren und $\alpha$-Hydroxysäuren, Isoprenoidlipide, Mono- oder Diglyceride, Sphingolipide, Poly-, Oligo- und Mononucleotide, Nucleoside, Antioxidationsmittel, Radikalfänger, Arzneistoffe, Antiseptika, Aroma- und Geschmackstoffe, Duftstoffe und Farbstoffe können als Akzeptor in der Fructosylierungsreaktion eingesetzt werden.

Als Vitamine können Ascorbinsäure (Vitamin C), Vitamine der Gruppe B wie Cyanocobalamin ($B_{12}$), Riboflavin, und Riboflavinmonophosphat ($B_2$), Pyridoxinhydrochlorid, Pyridoxol, Pyridoxal ($B_6$), Pantothensäure und Panthenol (Vitamin $B_5$) und Thiamin ($B_1$), ferner Retinol(Vitamin A), 3,4-Didehydroretinol (Vitamin $A_2$), und 3-Dehydroretinol sowie deren Aldehyde, Säuren und Ester; Ergocalciferol und ihre Verwandte (Vitamin $D_2$, $D_3$ und $D_4$), $\alpha$-Tocopherol (Vitamin E), Koagulationsvitamine wie Phytonadiol (Dihydrovitamin $K_1$), Menachinon-$H_2$ (Dihydrovitamin $K_2$), Menadiol (Dihydrovitamin $K_3$) und Vitamin $K_5$ in der Fructosylierungsreaktion eingesetzt werden.

Als Hormone sind Steroidhormone, wie Testosteron, Androsteron, Estradiol, Estriol, Corticosteron und Cortison, einsetzbar. Weitere Steroide wie Cholesterol sind auch in der Fructosylierungsreaktion einsetzbar. Weitere Hormone, wie Prostaglandine (Prostaglandine E1, E2 und F), Peptidhormone wie Ocytocin, Vasopressin, Glucagon, Serotonin und Insulin, Glycoproteine wie LH ("Luteinizing Hormone") und FSH ("Follicle-stimulating Hormone") können ebenfalls in der Fructosylierungsreaktion eingesetzt werden.

Als Akzeptoren können auch neurologische Botensubstanzen wie Adrenalin, Noradrenalin, Dopa (3-Hydroxytyrosin) und Dopamin, Aminosäuren, wie Serin, Threonin, und Tyrosin, Serin-, Threonin- und Tyrosin-enthaltende Peptide und Proteine, Hydroxysäuren und $\alpha$-Hydroxysäuren wie Milchsäure, $\alpha$-Galakturonsäure, 4-Hydroxybutansäure, 3-Hydroxybutansäure sowie Sphingolipide wie Ceramide, Glycoceramide und Sphingomyelin eingesetzt werden.

Als Akzeptor können auch Poly-, Oligo- und Mononucleotide, wie Ribonucleinsäure und Desoxyribonucleinsäure, Adenosinmono-, -di- und -triphosphat, cyclisches Adenosinmonophosphat, Nucleoside, Purin- und Pyrimidinbasen, wie Guanin, Hypoxanthin, Cytosin, Uracil und Thymin, eingesetzt werden. Analoga dieser Substanzen wie Zidovudin, Acyclovir und Cytarabin, die eine Antimetabolitwirkung besitzen, können ebenfalls eingesetzt werden.

Weitere einsetzbare Akzeptoren sind Substanzen, die ein oder mehrere aromatische hydroxylierte Ringe enthalten. Diese sind zum Beispiel Phenol, Salicylsäure, Hydrochinon, Arbutin, Pyrocatechol (Brenzcatechin), Resorcin, Phloroglucin, Pyrogallol, Cresol, Catechine, Tanninsäure und Bioflavonoide wie Quercetin und Luteolin. Weitere Beispiele sind Geschmacksstoffe wie Vanillin, Vanillinsäure und Ethylvanillin, UV-Filter wie Esculetin, Farbstoffe wie Kermesinsäure, Konservierungsmittel wie Ferulinsäure, Methyl-, Ethyl- und Propylparaben, Haarschutzmittel wie Folescutol sowie Arzneistoffe wie Alkaloide (z.B. Morphin, Cephaelin), Haemostatika (z.B. Adrenalon), beta-Agonisten (Epinephrine, Fenoterol), Antibiotika (Cloxyquin, Dichlorophen, Iodochinol), Analgetika (Paracetamol, Gentisinsäure), Antikrebsmittel (Mitoxantrone), Adrenergika (Octopamine) und Antihypertensive (Methyldopa).

Sekundäre Metabolite aus Pflanzen oder Mikroorganismen wie Kojisäure, Menthol, Thymol, Dihydroxyaceton und $\alpha$-Bisabolol können auch als Akzeptoren in der Fructosylierungsreaktion eingesetzt werden.

Der Donator und der Akzeptor der Fructosylierungsreaktion können in einer wäßrigen Lösung eingesetzt werden. Vorteilhaft ist aber auch die Anwendung von organischen Lösungsmitteln. Dadurch können wasserunlösliche Akzeptoren gelöst werden. Ein weiterer Vorteil des Einsatzes von organischen Lösungsmitteln ist die Möglichkeit, den Wassergehalt und die Wasseraktivität zu kontrollieren, was eine bessere Lenkung der Fructosylierungsreaktion ermöglicht. Die unerwünschte Fructosylierung des Wassers (Hydrolyse der Saccharose) wird dadurch gehemmt. Ebenfalls kann dadurch die unerwünschte Fructosylierung der Saccharose und der Glucose bei geringer Saccharidkonzentration gehemmt werden.

Die Reaktion kann in Einphasenlösungssystemen oder in Mehrphasenlösungssystemen eingesetzt werden. Mit Mehrphasensystemen können auch Tenside den Stoffaustausch verbessern. Beim Einsatz von Mehrphasensystemen kann das Enzym an der Grenzfläche wirken.

Die Auswahl der Lösungsmittel beruht auf folgenden Kriterien:

- Die Saccharose muß darin löslich sein
- Der Akzeptor muß darin löslich sein
- Das Enzym muß im Lösungssystem aktiv und stabil sein
- Das Lösungsmittel darf selber keine oder aber nur eine geringe Wirkung als Akzeptor besitzen.

Die Reaktion kann beispielsweise in Anwesenheit von polaren Lösungsmitteln, wie Aceton, Acetonitril, Dimethylformamid, Dimethylacetamid, Glyme, Methylethylketon oder Dioxan durchgeführt werden. Apolare Lösungsmittel wie Hexan können auch eingesetzt werden.

Die pH- und Temperaturbedingungen sowie die Wasseraktivität werden im günstigsten Bereich eingestellt, in dem die Fructosyltransferase aktiv und stabil ist und in dem die Reaktionsmittel löslich und stabil sind.

Die Fructosyltransferase wird dann zum Medium zugesetzt. Sie kann mikrobiell aus einer Vielfalt von Pilzen, wie *Aspergillus niger* und *Aureobasidium pullulans*, und Bakterien, wie *Bacillus subtils*, *Erwinia herbicola* und *Zymomonas mobilis,* gewonnen werden. Immobilisierte Fructosyltransferase und ganze Zellen sind auch zum erfindungsgemäßen Verfahren geeignet. Beim Einsatz von organischen Lösungsmitteln kann das Enzympräparat günstigerweise durch Lyophilisierung oder Lösungsmittelaustausch so getrocknet werden, daß es vom Lösungsmittel effizient benetzt Wird. Dies hat den Vorteil, daß die Ausgangsstoffe der Reaktion an das aktive Zentrum des Enzyms gelangen können.

Die Reaktionslösung wird dann inkubiert. Die Inkubationszeit hängt davon ab, welche Enzymmenge eingesetzt wird und welche Hemmungseffekte durch die Mediumkomponente auftreten. Geeignete Enzymmengen betragen 0,5-50 U/ml und geeignete Reaktionszeiten betragen 2-96 h. In Fällen von Multiphasensystemen muß das Reaktionsmedium während der Inkubation so gemischt werden, daß die Stoffübergänge zwischen den Phasen gewährleistet werden. Die Reaktion kann günstig in offenen Systemen betrieben werden, wobei Zugabe von Ausgangsstoffen bzw. Abgabe von Produkten während der Reaktion vorgenommen werden.

Nach der Inkubation werden die hergestellten Fructosylverbindungen durch bekannte Verfahren wie Chromatographie mit Ionenaustauschern, Ausfällung durch Alkohol oder Salze, Flashchromatographie und/oder Kristallisierung aus dem Reaktionsmedium extrahiert und gereinigt. Die Fructosylverbindungen werden nach Bedarf dann durch Kristallisierung, Sprühtrocknung oder Gefriertrocknung als Pulver dargestellt.

Diese neuen Fructosylverbindungen werden dann als solche oder in verschiedenen Präparaten eingesetzt. Abhängig von den Eigenschaften der als Akzeptor eingesetzten Rohmaterialien können durch dieses Verfahren Fructosylderivate hergestellt werden, die wie Antibiotika, Vitamine, Hormone, Aminosäuren, Antioxidationsmittel, Pestizide, neurologische Botenstoffe, sekundäre Metabolite aus Pflanzen oder Mikroorganismen, Aminosäuren, Peptide, Proteine, Hydroxysäuren und $\alpha$-Hydroxysäuren, Isoprenoidlipide, Mono- oder Diglyceride, Sphingolipide, Poly-, Oligo- und Mononucleotide, Nucleoside, Antioxidationsmittel, Radikalfänger, Arzneistoffe, Antiseptika, Aroma- und Geschmacksstoffe, Duftstoffe und Farbstoffe als Akzeptor in der Fructosylierungsreaktion wirken können. Die Fructosylverbindungen der Erfindung besitzen im Vergleich zu den Startmaterialien verbesserte Eigenschaften bezüglich Löslichkeit, Diffusionskoeffizient, Stabilität, Metabolisierungsgeschwindikeit. Dieses Verfahren kann zum Beispiel zur Vektorisierung von Arzneimitteln angewandt werden.

Die verbesserten Eigenschaften der erfindungsgemäßen Fructosylderivate sind auf die Kombination der Eigenschaften des Akzeptormoleküls mit den Eigenschaften der Fructosylgruppe zurückzuführen. So wurde gefunden, daß in vielen Fällen nicht nur die Wirksamkeit der erfindungsgemäßen Fructosyl-Akzeptor-Verbindung im Vergleich zum unfructosylierten Akzeptormolekül potenziert ist, sondern auch die neuen erfindungsgemäßen Verbindungen neue Eigenschaften und ein neues Wirkungsprofil aufweisen.

Die erfindungsgemäßen Verbindungen eignen sich insbesondere als Wirkstoffe zur topischen Anwendung, z. B. in kosmetischen Mitteln.

So kann durch die Verwendung einer geeigneten Fructosyl-Akzeptor-Verbindung eine verbesserte Verfügbarkeit des Wirkstoffes erreicht werden, was zum Beispiel auf die verbesserte Löslichkeit der Verbindung und somit der Bioverfügbarkeit des Wirkstoffs zurückzuführen ist, bzw. durch den langsameren Abbau der Verbindung auf der Haut, wodurch eine langanhaltende Pflege ermöglicht wird.

Auch können beispielsweise durch die Verwendung einer geeigneten Fructosyl-Akzeptor-Verbindung die üblichen Nebenwirkungen eines Wirkstoffes, z.B. die durch $\alpha$-Hydroxysäuren verursachten Nebenwirkungen erheblich vermindert werden.

Desweiteren eignen sich derartige die erfingungsgernäßen Verbindungen enthaltende Mittel insbesondere zur Förderung oder Wiederherstellung der Mikroflora der Haut, wobei das Wachstum von nicht erwünschten Mikroorganismen verhindert wird.

Aufgrund ihrer immunmodulatorischen Eigenschaften, bzw. entzündungshemmenden Wirkungen eignen sich derartige Mittel auch insbesondere zur Vorbeugung und zur Linderung von Hautreizungen.

Die Erfindung wird durch nachfolgende Ausführungsbeispiele erläutert.

Ausführungsbeispiele

Beispiel 1: Ascorbinsäure als Akzeptor

Zur Bildung von Fructosylverbindungen wurde die Fructosyltransferase (Levansaccharase) aus dem Bakterienstamm *Microbacterium laevaniformans* (DSM 20140) hergestellt. Ein Kulturmedium (Volumen: 160 ml in einer 500 ml Kolbenflasche mit Hefeextrakt, 12,5 g/l, Caseinpeptone, 12,5 g/l; Saccharose, 50 g/l) und eine Phosphatpufferlösung (Volumen: 20 ml mit $Na_2HPO_4$, 80 g/l; $NaH_2PO_4$, 40 g/l) wurden vorbereitet, bei pH 7,3 eingestellt und bei 121 °C 20 min autoklaviert. Nach Abkühlung wurde die Phosphatlösung der Nährlösung steril zugegeben. Diese gepufferte Nährlösung (180 ml) wurde dann mit 20 ml einer 24 h gewachsenen Vorkultur von *Mircrobacterium laevaniformans* angeimpft.

Die Kultur wurde dann bei 30-32 °C und 250 u/min auf einer rotierenden Schüttelmaschine inkubiert. Nach 24 Stunden Inkubation betrugen der pH-Wert 5,4 und die optische Dichte (Extinktion bei 600 nm) der Kultur 3,5. Zu der Fermentationslösung wurden 67 g Polyethylenglycol 1500 bei 10 °C unter Rühren hinzugegeben. Diese Lösung wurde dann bei 4200 g und 6 °C 30 min zentrifugiert. Der Überstand wurde verworfen und die levansaccharasereiche untere Phase mit 15 ml Natriumphsophatpuffer (pH 6,5; 50 mM) suspendiert. Dieses Enzympräparat wurde so in Fructosyltransferreaktionen als Levansaccharase eingesetzt.

Die Levansaccharaseaktivität wurde wie folgt bestimmt. Das Präparat wurde in Natriumphosphat 50mM pH 6,0 1:10 verdünnt. Ein Medium mit 250 μl Saccharose 400 g/l; 50 μl Natiumphosphatpuffer 1 M pH 6,0; 500 μl Wasser und 200 μl unverdünntes Enzympräparat wurden bei 30 °C inkubiert. Eine 50 μl -Probe wurde jede 5. Minute über 20 min. gezogen und mit 200 μl $Na_2CO_3$ 0,1 M verdünnt. In diesen letzteren Lösungen und in einer Glucosestandardreihe (0-4 g/l) wurde die Glucosekonzentration mit dem Glucose-Trinderreagenz (Sigma) gemessen. Eine Levansaccharaseeinheit (U) wurde definiert als die Enzymmenge, die ein Mikromol Glucose unter den Messbedingungen pro Minute freisetzt. Das Levansaccharasepräparat aus *Microbacterium laevaniformans* enthielt 35 U/ml.

Zur enzymatischen Fructosylierungsreaktion wurde ein Medium mit Saccharose, 1 g; Ascorbinsäure, 0,5 g; $Na_2CO_3$, 0,1 g; Wasser, 2,9 g vorbereitet. Während der Lösung der Bestandteile des Mediums wurde $CO_2$ freigesetzt, was eine Gasbildung verursachte und der Neutralisierung von Ascorbinsäure durch $Na_2CO_3$ entsprach. Das Medium hatte einen pH-Wert von 4,6. Die Fructosylierungsreaktion wurde durch Zugabe von 0,5 ml Levanssacharase aus *Microbacterium laevaniformans* begonnen und das Medium bei 30 °C über 15 h inkubiert. Eine 250 μl-Probe des Reaktionsmediums wurde nach 5 min und nach 15 h Inkubationszeit genommen und mit 4750 μl 0,1 N $H_2SO_4$ 1:20 verdünnt. Diese Lösungen wurden durch HPLC (Säule : BIORAD HPX 87-H, Eluent : 0.01 N $H_2SO_4$, Detektion: Brechungsindexdetektor und Diodenarraydetektor) analysiert. Die feste Phase der Säule ist ein Polystyren-Divinylbenzen-Ionenaustauschharz mit -$SO_3$-Gruppen als aktive Funktionen. In diesem chromatographischen System werden die Verbindungen in der folgenden Reihenfolge eluiert: Salze und Makromoleküle (Polysaccharide), Trisaccharide, Disaccharide und Monosaccharide.

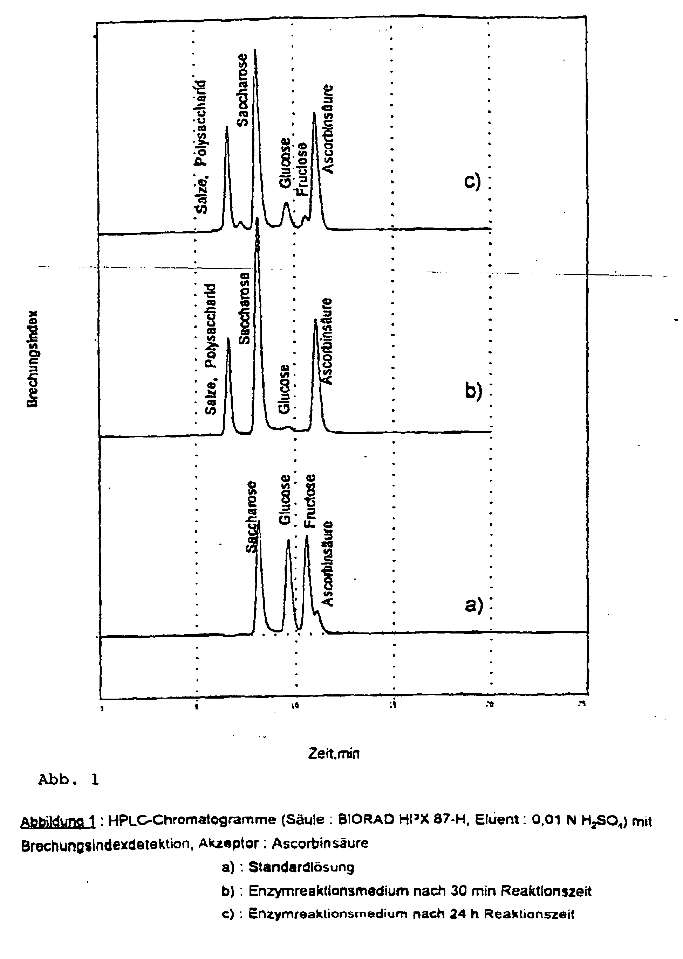

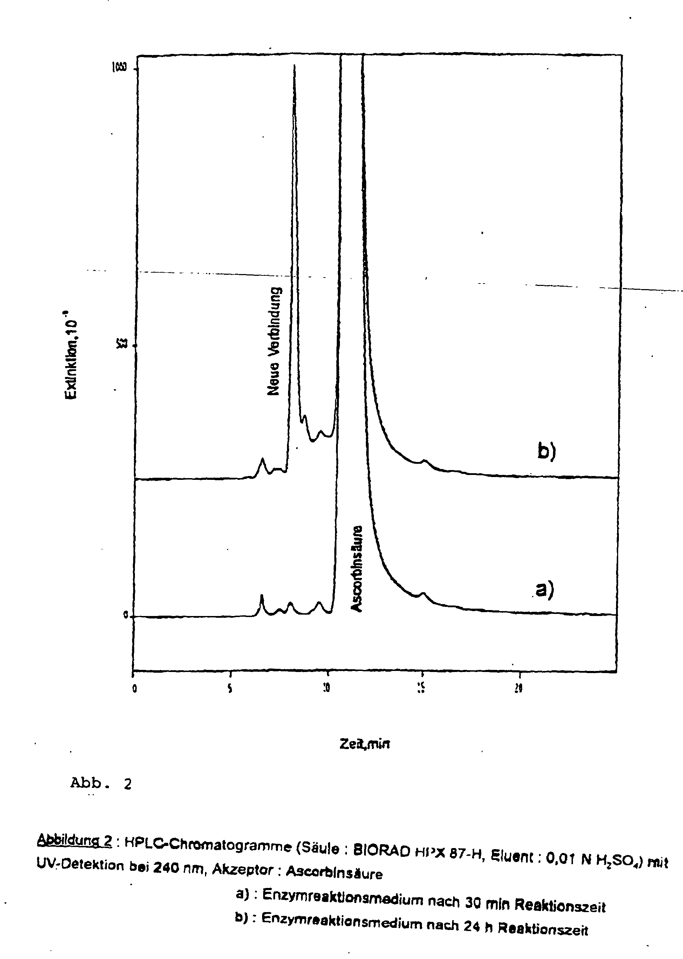

Die Chromatogramme aus der Brechungsindexdetektion am Anfang und am Ende der Reaktion werden in der Abbildung 1 dargestellt. Durch Vergleich mit dem Standardchromatogramm sind Saccharose, Glucose, Fructose und Ascorbinsäure im Reaktionsmedium zu erkennen. Durch die UV-Detektion bei 240 nm (Abbildung 2) wird die Ascorbinsäure als Chromophor identifiziert. Die starke Extinktion mit einem Maximum bei 245 nm ist für Ascorbinsäure charakteristisch und wird durch das konjugierte π-Elektronensystem der Ascorbinsäure hervorgerufen.

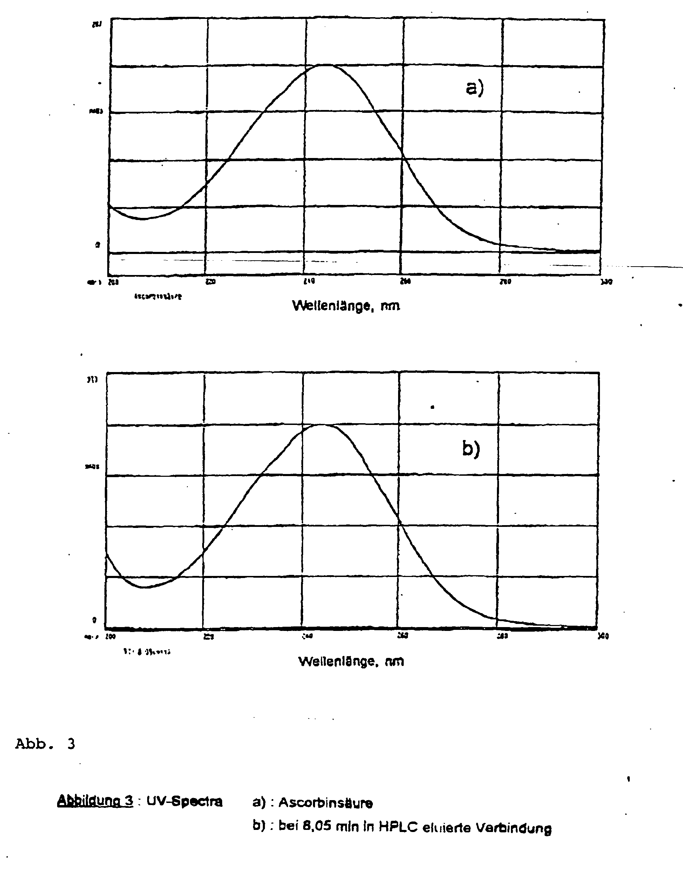

Während der enzymatischen Reaktion mit der Fructosyltransferase entsteht eine Verbindung, die im Gegensatz zu den Zuckern wie die Ascorbinsäure bei 240 nm das UV-Licht absorbiert. Diese Verbindung (Retentionszeit: 8,05 Min) verhält sich chromatographisch wie ein Disaccharid (z.B. Saccharose-Retentionszeit: 8,14 min). Sie hat dasselbe UV-Spektrum wie die Ascorbinsäure (Abbildung 3). Diese Verbindung besitzt die Eigenschaften von Ascorbinsäurefructosid.

Beispiel 2: Arbutin als Akzeptor

Zur Anreicherung von Levansaccharase aus *Bacillus subtilis* (DSM 10) wurden ein Kulturmedium (Volumen: 1,9 l mit Hefeextrakt 3g; Caseinpepton 30 g; Fleischextrakt 3g; $(NH_4)_2HPO_4$ 60 g; Saccharose 140 g; Tween 80 5g) und eine Salzlösung (Volumen: 0,2 1 mit $MgSO_4$ x $7H_2O$ 600 mg; KCl 1000 mg; $CaCl_2$ x $2H_2O$ 600 mg; $MnSO_4$ x $H_2O$ 30 mg; $FeSO_4$ x $7H_2O$ 30 mg; NaCl 30 mg) vorbereitet. Der pH-Wert des Mediums wurde mit Phosphorsäure auf 7,3 eingestellt. Das Kulturmedium wurde bei 120 °C 20 min in einem 5-l-Fermenter autoklaviert. Die Salzlösung wurde mit Salzsäure auf pH 2 eingestellt und bei 121 °C 20 min autoklaviert. Nach Abkühlung wurde die Salzlösung steril zu dem Kulturmedium gegeben.

Die Temperatur der Kultur wurde bei 30 °C und die Belüftungsrate bei 180 1/h gehalten. Währen der ersten 12 h der Fermentation fiel der pH-Wert auf 6,5. Er wurde dann durch Zugabe einer 12,5%igen Ammoniaklösung bei diesem Wert gehalten. Die Rührerdrehzahl wurde am Anfang der Fermentation bei 600 u/min eingestellt und danach nach Bedarf bis auf 1200 u/min erhöht, damit die Konzentration des gelösten Sauerstoffs nicht unter 20 % der der Luftsättigung fiel. Nach Bedarf wurde Antischaummittel (M30 Silikon-Antischaumemulsion, Sigma) dem Fermentationsmedium zugesetzt.

Nach 15 h Fermentation wurden 600 ml innerhalb von 15 h einer Saccharoselösung (40 % w/w) schrittweise zum Fermentationsmedium hinzugegeben. Die Fermentation wurde nach 30 h beendet. Nach Zentrifugation der Fermentationslösung für 30 min bei 2000 g wurde der Überstand durch Filterschichten filtriert. In dem Filtrat (2400 g) wurden 800 g Polyethylenglycol 1500 bei 10 °C gelöst.

Dadurch wurde die Levansaccharase ausgefällt. Die daraus resultierende Suspension wurde über Nacht bei 10 °C stehengelassen. Die sich gebildeten zwei Phasen wurden durch Dekantieren voneinander getrennt. Anschließend wurde die untere levansaccharasereiche Phase (250 ml) mit 370 ml 50 mM Natriumphosphatpuffer (pH 6,0) verdünnt. In dieser Lösung (690 g) wurden 230 g PEG 1500 bei 10 °C gelöst. Die resultierende Suspension wurde 30 min bei 10 °C und 4200 g zentrifugiert. Die levansaccharasereiche untere Phase wurde mit 50 mM Natriumphosphatpuffer (pH 6,0) 1:2 verdünnt. Dieses Präparat (212 ml) enthielt 13 U/ml Levansaccharase und wurde für die Fructosyltransferreaktionen eingesetzt.

Zur enzymatischen Fructosylierungsreaktion wurde ein Medium mit Saccharose 100 mg; Arbutin 100 mg; 1 M Natriumphosphatpuffer pH 6,0 25 μl; Wasser 225 μl vorbereitet. Die Bestandteile des Mediums wurden durch Erhitzen auf 70 °C gelöst. Das Medium wurde dann bei 50 °C gehalten. Der pH-Wert des Mediums, durch pH-Indikatorpapier bestimmt, betrug 6 - 6,5. Die Fructosylierungsreaktion wurde durch Zugabe von 50 μl Levansaccharase aus *Bacillus subtilis* begonnen und die Mischung bei 50 °C über 19 h inkubiert. Eine gewogene Probe (ca. 50 mg) des Reaktionsmediums wurde nach 19 h Inkubation abgenommen und 5 min bei 100 °C gehalten. Die Probe wurde anschließend mit ca. 950 $\mu$l Wasser 1:20 verdünnt. Davon wurden 200 $\mu$l entnommen und mit 800 $\mu$l 0,1 N $H_2SO_4$ 1:5 verdünnt. Als Kontrolle diente ein Ansatz mit thermisch (20 min bei 100 °C) denaturiertem Enzym. Diese Lösungen wurden durch HPLC (Säule: BIORAD HPX 87-H, Eluent: 0,01 N $H_2SO_4$, Detektion: Brechungsindexdetektor und Diodenarraydetektor) wie im Beispiel 1 analysiert.

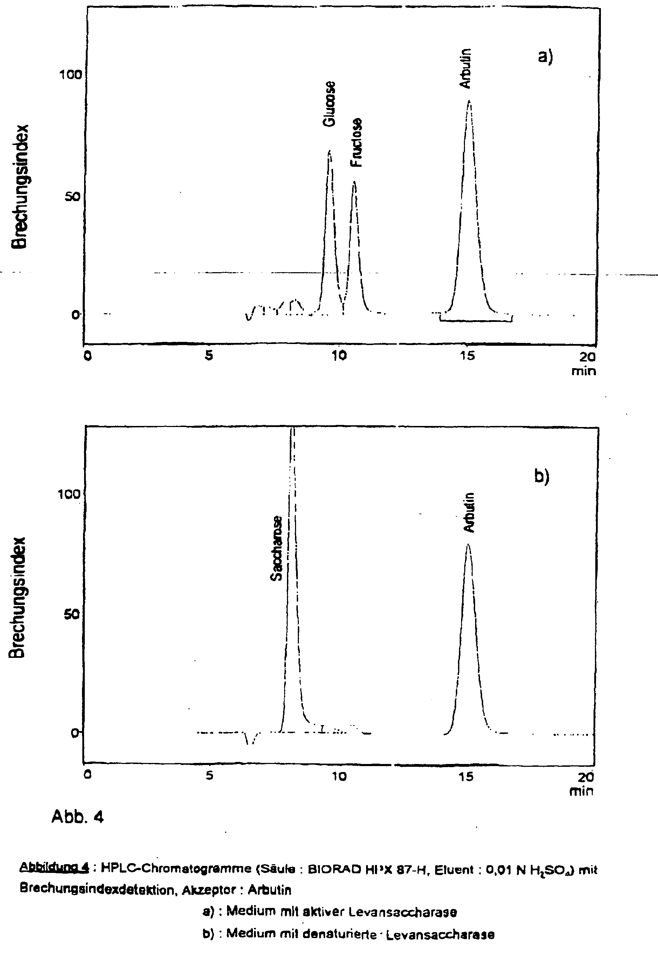

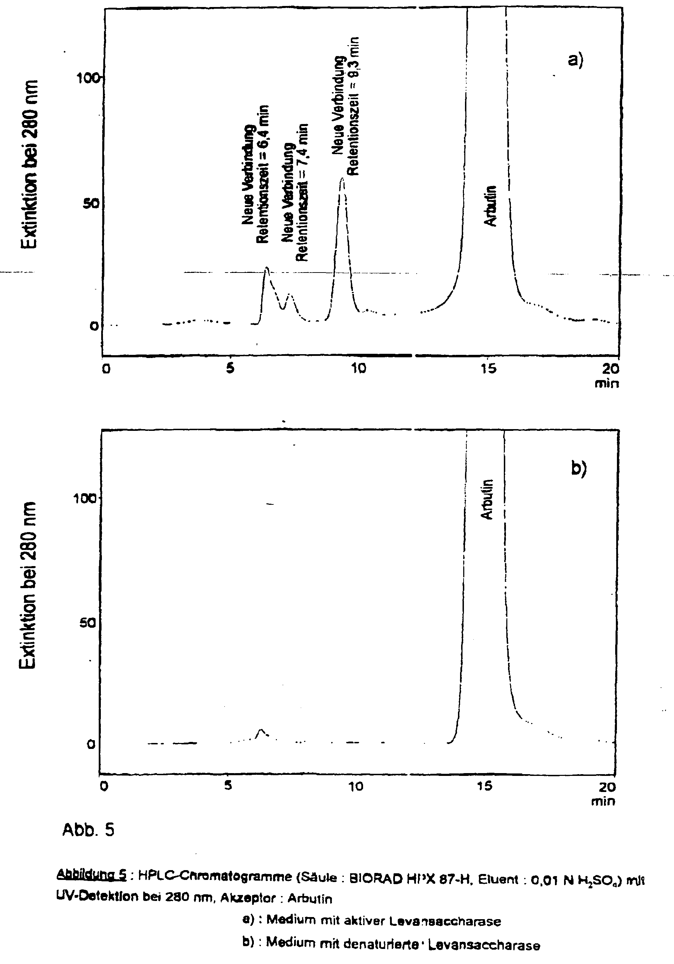

Die Chromatogramme aus der Brechungsindexdetektion mit aktiver und mit denaturierter Levansaccharase sind in der Abbildung 4 dargestellt. Darin sind Saccharose, Glucose, Fructose und Arbutin zu erkennen. Durch UV-Detektion bei 280 nm (Abbildung 5) wurde das Arbutin als Chromophor identifiziert. Die starke Extinktion mit einem Maximum bei 280 nm ist für Arbutin charakteristisch und wird durch den aromatischen Ring des Arbutins hervorgerufen.

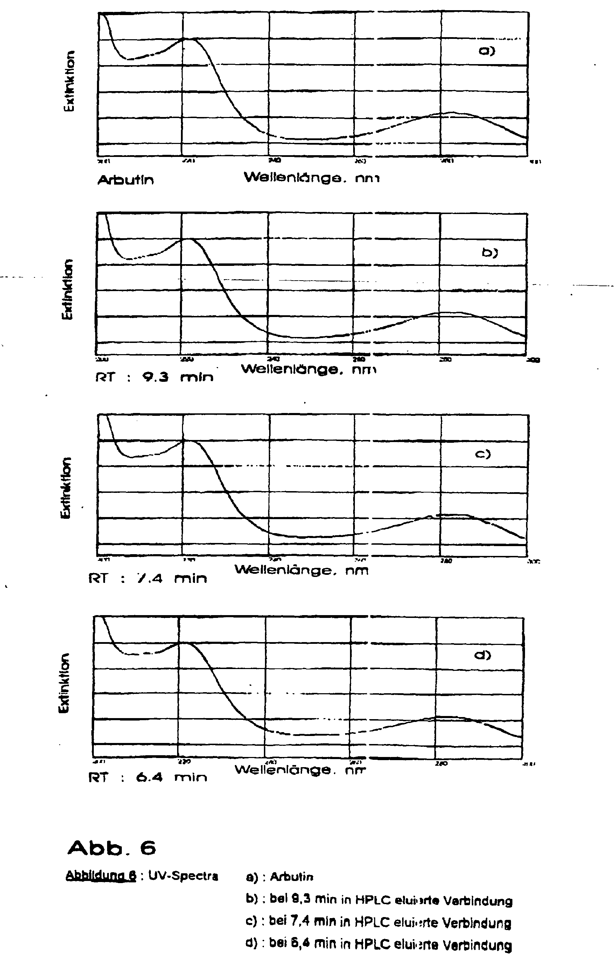

Durch die enzymatische Reaktion mit der aktiven Fructosyltransferase entstehen mehrere Verbindungen, die wie das Arbutin bei 280 nm das UV-Licht absorbieren. Diese neuen Verbindungen (Retentionszeiten: 9,3 min, 7, 4 min und 6,4 min) besitzen geringere Retentionszeiten als Arbutin, was auf eine größere Anzahl an Monosaccharidresten hinweist. Sie weisen aber das für Arbutin charakteristische UV-Spektrum auf (Abbildung 6). Diese gebildeten Verbindungen zeigen somit das chromatographische Verhalten sowie die spektroskopischen Eigenschaften von fructosylierten Arbutinderivaten.

Beispiel 3: Galacturonsäure als Akzeptor

Die Levansaccharase wurde gemäß Beispiel 2 gewonnen. Zum Einsatz von Galacturonsäure als Akzeptor wurde ein Medium mit Saccharose 100 mg; Galacturonsäure 100 mg; Natriumcarbonat 25 mg, 1 M Natriumphosphatpuffer pH 6,0 25 $\mu$l; Wasser 205 $\mu$l; Wasser 205 $\mu$l vorbereitet. Die Galacturonsäure und das Natriumcarbonat wurden zuerst gelöst. Der pH-Wert (durch pH-Indikatorpapier bestimmt) lag bei 4,5. Die Saccharose wurde dann im Medium gelöst. Durch Zugabe von 50 $\mu$l Levansaccharase aus *Bacillus subtilis* wurde die Fructosylierungsreaktion begonnen. Das Reaktionsgemisch wurde bei 40 °C 19 h inkubiert. Eine gewogene Probe (ca. 50 mg) des Reaktionsmediums wurde nach 19 h Inkubation genommen und 5 min bei 100 °C gehalten. Die Probe wurde anschließend mit ca. 950 $\mu$l Wasser 1:20 verdünnt. 50 $\mu$l von dieser Lösung wurden mit 450 $\mu$l Wasser 1:10 verdünnt. Davon wurden wiederum 50 $\mu$l abpipettiert und mit 950 $\mu$l Wasser 1:20 verdünnt. Als Kontrolle diente ein Ansatz mit thermisch (20 min bei 100 °C) denaturiertem Enzym.

Die Lösungen wurden durch Ionenchromatographie mit einer DIONEX-Carbopack PA-1 Säule und amperometrischer Detektion analysiert. Die Elution erfolgte mit 0,1 M NaOH und einem Natriumacetat-Gradienten. Der Gradient wurde aus einer 0,1 M NaOH Lösung (A) und einer 0,1 M NaOH Lösung mit 1 M NaOAc (B) folgendermaßen angelegt:

| Zeit, min | A, % | B, % |
|---|---|---|
| 0 | 98 | 2 |
| 5 | 91 | 9 |
| 20 | 66 | 34 |

Bei einem hohen pH-Wert wurden die Alkoholfunktionen der Zucker partiell als Alkoholate ionisiert. Die Zucker verhalten sich dabei anionisch und werden durch die Anionenaustauschersäule zurückgehalten. Je mehr Alkoholfunktionen ein Molekül trägt, desto stärker ist die Rückhaltung dieses Moleküls auf der Säule. Die Zucker eluieren daher mit steigendem Polymerisationsgrad langsamer. Darüber hinaus hängt die Ioinisierbarkeit der OH-Gruppe von der Gesamtgestalt des Moleküls ab. Je ionisierbarer die OH-Gruppen eines Zuckers sind, desto stärker wird der Zucker über die Säule zurückgehalten. Eine Trennung von verschiedenen Zuckern mit gleichem Polymerisationsgrad ist dadurch möglich. Die Säuren sind in diesem System noch stärker ionisiert als die Zucker und werden entsprechend stärker zurückgehalten.

Die Zucker und Zuckersäuren wurden durch pulsierende Amperometrie mit einer Goldelektrode detektiert. Dabei werden sie bei hohem pH-Wert elektrochemisch oxidiert, und das entsprechende zeitliche Auftreten der Oxidationsströme wird bestimmt.

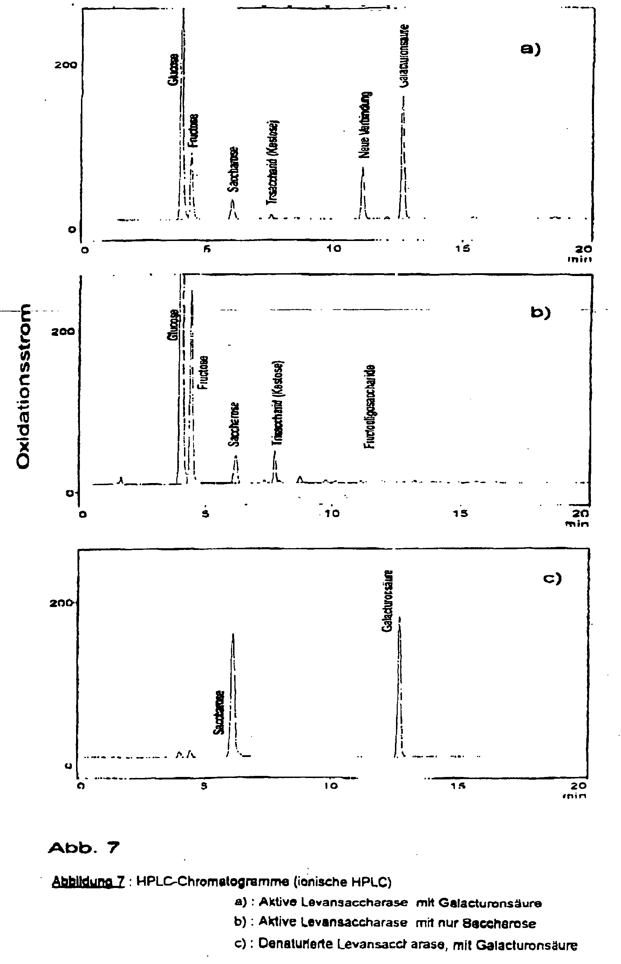

Die Chromatogramme der ionischen HPLC mit aktiver und mit denaturierter Levansaccarase sind in der Abbildung 7 dargestellt. Saccharose, Glucose, Fructose und Galacturonsäure wurden im Reaktionsgemisch identifiziert. Als zweite Kontrolle diente ein Reaktionsansatz mit aktiver Levansaccharase, in dem Galacturonsäure durch Saccharose ersetzt wurde. In dem entsprechenden Chromatogramm (Abbildung 7b) sind ein Trisaccharid und Oligosaccharide anhand ihrer Retentionszeiten zu erkennen. Sie resultieren aus der Fructosylierung von Saccharose.

Durch die enzymatische Reaktion mit der aktiven Fructosyltransferase entsteht nur in Anwesenheit von Galacturonsäure eine neue Verbindung. Sie wird stärker als die in Anwesenheit von Saccharose gebildeten Saccharide zurückgehalten, was auf eine höhere Affinität für die Anionenaustauschersäule schließen läßt. Diese Verbindung ist eine aus Galacturonsäure gebildete fructosylierte organische Säure. Sie besitzt die Eigenschaften von Galacturonsäurefructosid.

Beispiel 4: Gluconsäure als Akzeptor

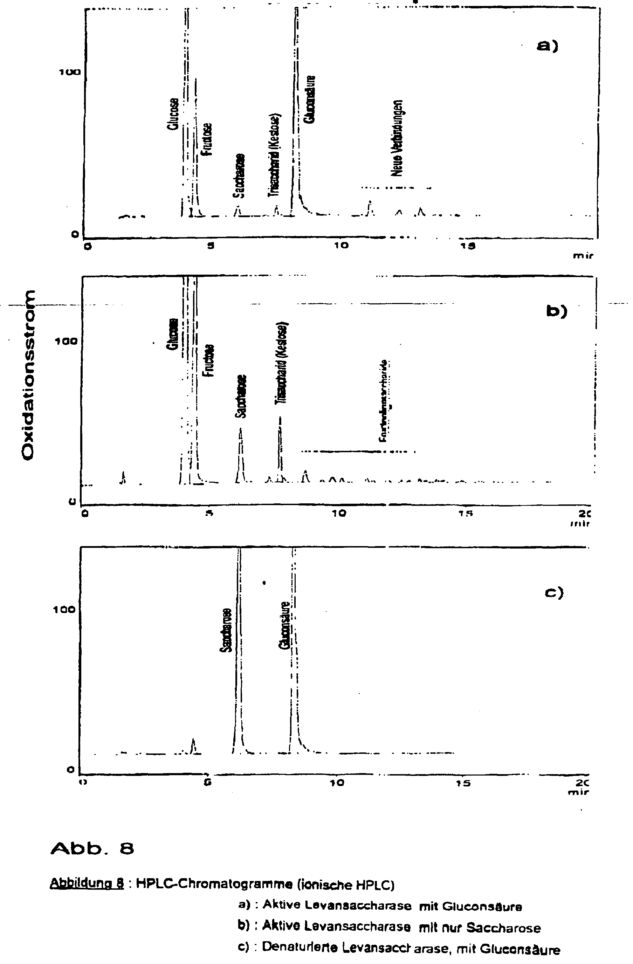

Die Levansaccharase wurde gemäß Beispiel 2 gewonnen.
Die Gluconsäure wurde unter den Bedingungen des Beispiels 3 wie Galacturonsäure als Akzeptor eingesetzt. Die Reaktionsprodukte wurden, wie in Beispiel 3 beschrieben, analysiert.
Die Chromatogramme der ionischen HPLC mit aktiver und mit denaturierter Levansaccharase sind in Abbildung 8 dargestellt. Saccharose, Glucose, Fructose und Gluconsäure wurden im Reaktionsgemisch identifiziert. Als zweite Kontrolle diente ein Reaktionsansatz mit aktiver Levansaccharase in dem die Gluconsäure durch Saccharose ersetzt wurde. In dem entsprechenden Chromatogramm (Abbildung 8b) sind ein Trisaccharid und Oligosaccharide anhand ihrer Retentionszeiten zu erkennen. sie resultieren von der Fructosylierung von Saccharose.
Durch die enzymatische Reaktion mit der aktiven Fructosyltransferase entstehen nur in Anwesenheit von Gluconsäure meherer neue Verbindungen. Sie werden stärker als die in Anwesenheit von Saccharose gebildeten Saccharide auf der Säule zurückgehalten, was auf eine höhere Afffinität für die Anionenaustauschersäule schließen läßt. Diese Verbindungen sind aus Gluconsäure gebildete fructosylierte organische Säuren. Sie besitzen die Eigenschaften von Gluconsäurefructosiden.

**Patentansprüche**

1.  Verfahren zur enzymatischen Herstellung von Fructosylverbindungen durch die Einwirkung einer Fructosyltransferase auf eine Lösung von Saccharose und einem Akzeptor, wobei der Akzeptor ein Vitamin, Hormon, neurologischer Botenstoff, eine Aminosäure, ein Peptid, Protein, eine Hydroxysäure, $\alpha$-Hydroxysäure, ein Isoprenoidlipid, Mono- oder Diglycerid, Sphingolipid, Poly-, Oligo- oder Mononucleotid, Nucleosid, Antioxidationsmittel, Radikalfänger, Arzneistoff, Antiseptikum, Aroma- oder Geschmackstoff, Duftstoff, Farbstoff, Kojisäure, Menthol, Thymol, Dihydroxyaceton oder $\alpha$-Bisabolol ist und die enzymatisch aufgebauten Bindungen Fructosidbindungen sind.

2.  Verfahren nach Anspruch 1, **dadurch gekennzeichnet, dass** die Fructosyltransferase Levansaccharase (E.C. 2.4.1.10) ist.

3.  Verfahren nach Anspruch 1 oder 2, wobei der Akzeptor mindestens eine OH-Gruppe umfasst.

4.  Verfahren nach Anspruch 1-3, **dadurch gekennzeichnet, dass** der Akzeptor ein Vitamin darstellt.

5.  Verfahren nach Anspruch 1-3, **dadurch gekennzeichnet, dass** der Akzeptor ein oder mehrere aromatische hydroxylierte Ringe enthält.

6.  Verfahren nach Anspruch 1-3, **dadurch gekennzeichnet, dass** der Akzeptor Kojisäure, Menthol, Thymol, Dihydroxyaceton oder $\alpha$-Bisabolol ist.

7.  Fructosylverbindungen, herstellbar gemäß einem der vorstehenden Ansprüche.

8.  Fructosylverbindungen der allgemeinen Formel

    $$F_r\text{-}O\text{-}(F_r\text{-}O)_n\text{-}A$$

    wobei

    $F_r$ eine Fructofuranosylgruppe
    n eine ganze Zahl zwischen 0 und 8,
    O ein Sauerstoffatom und
    A ein Vitamin, Hormon, eine neurologische Botensubstanz, Kojisäure, Menthol, Thymol, Dihydroxyaceton, $\alpha$-Bisabolol, eine Aminosäure, ein Peptid, Protein, eine Hydroxysäure, $\alpha$-Hydroxysäure, ein Isoprenoidlipid, Mono- oder Diglycerid, Sphingolipid, Poly-, Oligo- und Mononucleotid, Nucleosid, Antioxidationsmittel, einen Radikalfänger, Arzneistoff, ein Antiseptikum, einen Aroma- und Geschmackstoff, Duftstoff oder Farbstoff darstellt.

9.  Fructosylverbindugn gemäß Anspruch 8, wobei die $F_r$-O-$F_r$-Bindungen (falls $n \geq 1$) und die $F_r$-O-A-Bindung ausschließlich β-Fructosid-Bindungen sind.

**10.** Kosmetische Mittel, enthaltend eine Fructosylverbindung gemäß Anspruch 8 oder 9.

**11.** Arzneimittel enthaltend eine Fructosylverbindung gemäß Anspruch 8 oder 9.

## Claims

**1.** Process for the enzymatic preparation of fructosyl compounds by the action of a fructosyl transferase on a solution of sucrose and an acceptor, where the acceptor is a vitamin, hormone, neurological messenger, an amino acid, a peptide, protein, a hydroxy acid, $\alpha$-hydroxy acid, an isoprenoid lipid, mono- or diglyceride, sphingolipid, poly-, oligo- or mononucleotide, nucleoside, antioxidant, free radical scavenger, medicinal substance, antiseptic, aromatic substance or flavouring, fragrance, colorant, kojic acid, menthol, thymol, dihydroxyacetone or $\alpha$-bisabolol, and the enzymatically assembled linkages are fructoside linkages.

**2.** Process according to Claim 1, **characterized in that** the fructosyl transferase is levansucrase (E.G.2.4.1.10).

**3.** Process according to Claim 1 or 2, where the acceptor comprises at least one OH group.

**4.** Process according to Claim 1-3, **characterized in that** the acceptor represents a vitamin.

**5.** Process according to Claim 1-3, **characterized in that** the acceptor comprises one or more aromatic hydroxylated rings.

**6.** Process according to Claim 1-3, **characterized in that** the acceptor is kojic acid, menthol, thymol, dihydroxyacetone or $\alpha$-bisabolol.

**7.** Fructosyl compounds which can be prepared according to any of the preceding claims.

**8.** Fructosyl compounds of the general formula

$$F_r\text{-}O\text{-}(F_r\text{-}O)_n\text{-}A$$

where

$F_r$ is a fructofuranosyl group
n is an integer between 0 and 8,
O is an oxygen atom and
A is a vitamin, hormone, a neurological messenger, kojic acid, menthol, thymol, dihydroxyacetone, $\alpha$-bisabolol, an amino acid, a peptide, protein, a hydroxy acid, $\alpha$-hydroxy acid, an isoprenoid lipid, mono- or diglyceride, sphingolipid, poly-, oligo- and mononucleotide, nucleoside, antioxidant, a free radical scavenger, medicinal substance, an antiseptic, an aromatic substance and flavouring, fragrance or colorant.

**9.** Fructosyl compounds according to Claim 8, where the $F_r$-O-$F_r$ linkages (if $n \geq 1$) and the $F_r$-O-A linkage are exclusively $\beta$-fructoside linkages.

**10.** Cosmetic compositions comprising a fructosyl compound according to Claim 8 or 9.

**11.** Medicaments comprising a fructosyl compound according to Claim 8 or 9.

## Revendications

**1.** Procédé de production enzymatique de composés de fructosyle par l'action d'une fructosyle transférase sur une solution de saccharose et d'un accepteur, l'accepteur étant une vitamine, une hormone, un messager neurologique, un acide aminé, un peptide, une protéine, un hydroxyacide, un alpha-hydroxyacide, un lipide isoprénoïde, un mono ou diglycéride, un sphingolipide, un poly-, oligo- ou mononucléotide, un nucléoside, un agent antioxydant, un piège à radicaux, une substance médicamenteuse, un antiseptique, un agent aromatique ou de sapidité, une substance odoriférante, un colorant, de l'acide kojique, du menthol, du thymol, de la dihydroxyacétone ou de l'alpha-bisabolol, et les liaisons générées par voie enzymatique étant des liaisons fructoside.

2. Procédé selon la revendication 1, **caractérisé en ce que** la fructosyle transférase est de la lévane saccharase (E.C.2.4.1.10).

3. Procédé selon la revendication 1 ou 2, dans lequel l'accepteur renferme au moins un groupe OH.

4. Procédé selon la revendication 1-3, **caractérisé en ce que** l'accepteur représente une vitamine.

5. Procédé selon la revendication 1-3, **caractérisé en ce que** l'accepteur contient un ou plusieurs cycles aromatiques hydroxylés.

6. Procédé selon la revendication 1-3, **caractérisé en ce que** l'accepteur est de l'acide kojique, du menthol, du thymol, de la dihydroxyacétone ou de l'alpha-bisabolol.

7. Composés de fructosyle, pouvant être produits selon l'une quelconque des revendications précédentes.

8. Composés de fructosyle de formule générale

$$F_r\text{-}O\text{-}(F_r\text{-}O)_n\text{-}A$$

dans laquelle

$F_r$ représente un groupe fructofuranosyle
n est un nombre entier compris entre 0 et 8,
O est un atome d'oxygène et
A représente une vitamine, une hormone, un messager neurologique, de l'acide kojique, du menthol, du thymol, de la dihydroxyacétone, de l'alpha-bisabolol, un acide aminé, un peptide, une protéine, un hydroxyacide, de l'alpha-hydroxyacide, un lipide isoprénoïde, un mono ou diglycéride, un sphingolipide, un poly-, oligo- et mononucléotide, un nucléoside, un agent antioxydant, un piège à radicaux, une substance médicamenteuse, un antiseptique, un agent aromatique et de sapidité, une substance odoriférante ou un colorant.

9. Composé de fructosyle selon la revendication 8, dans lequel les liaisons $F_r\text{-}O\text{-}F_r$ (si n $\geq$ 1) et la liaison $F_r\text{-}O\text{-}A$ sont exclusivement des liaisons $\beta$-fructoside.

10. Produits cosmétiques, contenant un composé de fructosyle selon la revendication 8 ou 9.

11. Médicaments contenant un composé de fructosyle selon la revendication 8 ou 9.

Abb. 1

Abbildung 1 : HPLC-Chromalogramme (Säule : BIORAD HPX 87-H, Eluent : 0,01 N H₂SO₄) mit Brechungsindexdetektion, Akzeptor : Ascorbinsäure

        a) : Standardlösung

        b) : Enzymreaktionsmedium nach 30 min Reaktionszeit

        c) : Enzymreaktionsmedium nach 24 h Reaktionszeit

Abb. 2

Abbildung 2 : HPLC-Chromatogramme (Säule : BIORAD HPX 87-H, Eluent : 0,01 N $H_2SO_4$) mit UV-Detektion bei 240 nm, Akzeptor : Ascorbinsäure

a) : Enzymreaktionsmedium nach 30 min Reaktionszeit

b) : Enzymreaktionsmedium nach 24 h Reaktionszeit

Wellenlänge, nm

Wellenlänge, nm

Abb. 3

Abbildung 3 : UV-Spectra    a) : Ascorbinsäure

b) : bei 8,05 min in HPLC eluierte Verbindung

Abb. 4

Abbildung 4 : HPLC-Chromatogramme (Säule : BIORAD HPX 87-H, Eluent : 0,01 N H₂SO₄) mit Brechungsindexdetektion, Akzeptor : Arbutin

a) : Medium mit aktiver Levansaccharase

b) : Medium mit denaturierte · Levansaccharase

Abb. 5

<u>Abbildung 5</u> : HPLC-Chromatogramme (Säule : BIORAD HI'X 87-H, Eluent : 0,01 N H$_2$SO$_4$) mit UV-Detektion bei 280 nm, Akzeptor : Arbutin

a) : Medium mit aktiver Levansaccharase

b) : Medium mit denaturierte· Levansaccharase

a)

Arbutin · Wellenlänge, nm

b)

RT : 9.3 min · Wellenlänge, nm

c)

RT : 7.4 min · Wellenlänge, nm

d)

RT : 6.4 min · Wellenlänge, nm

# Abb. 6

Abbildung 6 : UV-Spectra   a) : Arbutin

b) : bei 9,3 min in HPLC eluierte Verbindung

c) : bei 7,4 min in HPLC eluierte Verbindung

d) : bei 6,4 min in HPLC eluierte Verbindung

**Abb. 7**

Abbildung 7 : HPLC-Chromatogramme (ionische HPLC)

        a) : Aktive Levansaccharase mit Galacturonsäure

        b) : Aktive Levansaccharase mit nur Saccharose

        c) : Denaturierte Levansaccharase, mit Galacturonsäure

## Abb. 8

Abbildung 8 : HPLC-Chromatogramme (ionische HPLC)

        a) : Aktive Levansaccharase mit Gluconsäure

        b) : Aktive Levansaccharase mit nur Saccharose

        c) : Denaturierte Levansaccharase, mit Gluconsäure